(19) Europäisches Patentamt
European Patent Office
Office européen des brevets

(11) EP 3 818 995 A1

(12) EUROPEAN PATENT APPLICATION
published in accordance with Art. 153(4) EPC

(43) Date of publication:
**12.05.2021 Bulletin 2021/19**

(21) Application number: **18925220.8**

(22) Date of filing: **19.09.2018**

(51) Int Cl.:
***A61L 31/02*** (2006.01)

(86) International application number:
**PCT/CN2018/106402**

(87) International publication number:
**WO 2020/006882 (09.01.2020 Gazette 2020/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **04.07.2018 CN 201810720954**

(71) Applicant: **Zhuhai Ruizhan Biomaterials Co., Ltd.**
**Zhuhai, Guangdong 519000 (CN)**

(72) Inventor: **ZHANG, Wenfang**
**Wuxi, Jiangsu 214142 (CN)**

(74) Representative: **Dargiewicz, Joanna**
**JD&P Patent Attorneys**
**Joanna Dargiewicz & Partners**
**Ul. Mysliborska 93A/50**
**03-185 Warszawa (PL)**

# (54) DEGRADABLE SPIRAL COATED STENT WITH CONTROLLABLE GRADIENT, PREPARATION METHOD THEREFOR AND USE THEREOF

(57) Disclosed are a spiral coated stent with controllable gradient degradation, a preparation method thereof and an application thereof. The spiral coated stent with controllable gradient degradation is composed of a degradable medical polyurethane and a degradable magnesium alloy material, wherein the degradable medical polyurethane contains a following chemical structure: PCL-PEG-PCL, wherein a molecular weight of the PEG is 200 to 1,000 and the molecular weight of the PCL is 200 to 10,000, and the degradable magnesium alloy material is of a spiral stent structure; and physical properties of the spiral coated stent with controllable gradient degradation need to satisfy the following technical parameters that: a breaking strength needs to be no less than 1 N, a pressure resistance needs to be no less than 2 N, and a degradation characteristic of the magnesium alloy after surface treatment shows gradient degradation with different time.

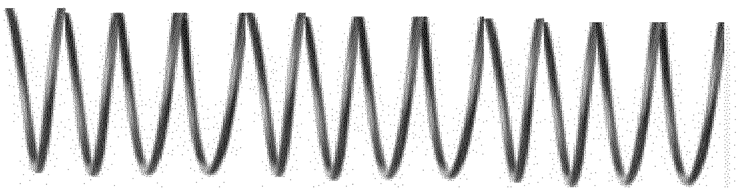

FIG. 1

## Description

## TECHNICAL FIELD

The disclosure belongs to the technical field of medical biomaterials and medical treatment, and particularly relates to a spiral coated stent with controllable gradient degradation, a preparation method thereof and an application thereof.

## BACKGROUND

Degradable stents may be used in various vessels in vivo, including natural body passages or body lumens, and further including artificial body openings and body lumens, such as bypass or ileostomy. For instance, the degradable stents include: coronary stents, intracranial stents, peripheral stents, splenic artery stents, intraoperative stents, heart valve stents, biliary stents, esophageal stents, intestinal stents, pancreatic stents, urethral stents, tracheal stents and ureteral stents. The research and application of the vascular stents is the most mature in clinic. It has been reported in literature that polylactic acid is used as a vascular stent of a drug-loading coating, and PLLA is also used to directly prepare the vascular stents through 3D printing or engraving.

The degradable stents include polymer material stents and degradable metal stents, wherein the degradable polymer materials include PLLA, PLA, PGA, PDO and PCL. The PLLA has good rigidity, flexibility, stability resistance and thermostability, and has been successfully applied to coating materials of metal stents and self-prepared into stents. However, aseptic inflammation caused by a local acidic environment due to degradation products thereof limits the PLLA to be applied as an implanted product.

Magnesium is an essential element in human metabolism, which is second only to potassium, sodium and calcium in a human body, and the magnesium in bone tissues accounts for about half of all the magnesium in the human body. Researches suggest that the magnesium is a cofactor of many enzymes and has stable DNA and RNA structures. The magnesium is kept between 0.7 mmol/L and 1.05 mmol/L through a kidney and intestines in vivo. The magnesium can stimulate growth of new bones and has good histocompatibility. The main disadvantage of the magnesium is low corrosion resistance. In a physiological environment of pH (7.4 to 7.6), the magnesium has a strong reduction effect, which leads to the loss of mechanical integrity before a tissue fully heals, and produces hydrogen that cannot be absorbed by the body in time. Magnesium-based materials used in the human body at an early stage, produced a large amount of gas after being implanted into the human body, which made the magnesium unable to be used in the human body. Therefore, it is of great practical significance to prepare magnesium-based alloys with controllable gradient degradation, so that the hydrogen generated in the degradation process of the magnesium can be metabolized by a tissue fluid. Recently, various magnesium alloy materials with different degradation and processability have also become research hotspots.

Materials of the degradable metal stents include magnesium alloy and iron alloy materials, wherein the magnesium alloy materials have gradually become a mainstream of degradable stent research due to excellent processability thereof. Because tissues and organs in different parts such as blood vessels, trachea and urethra have different requirements on performances of biomaterials for tissue engineering, it is very important to obtain a series of biomaterials with different mechanical properties, degradability and processability. Because of mechanical properties of a single material and acid-base environments produced by degradation, aseptic inflammation in vivo is caused. Therefore, it is a main orientation to adopt a better stent design technology and find more suitable composite polymer materials for in vivo implantation.

Many literatures have reported that various types of polyurethane materials have the features of good mechanical properties, biocompatibility, blood compatibility and easy processing, and have attractive prospects in the fields of drug sustained-release carriers, medical surgical materials, tissue engineering stents, or the like. However, due to the difficulty in controlling the synthesis process of the materials, the synthesis and evaluation of materials with controllable gradient degradation need a lot of basic research and experiments, so there is no medical polyurethane material with controllable gradient degradation on the market at present.

Degradable medical polyurethane is polymerized from four different compounds, and a specific reaction process is as follows: different initiators initiate polymer diols synthesized by different monomers, different diisocyanates react with the polymer diols, and then different chain extenders further react and extend a chain of an obtained product. Therefore, the change of any one of the raw materials will lead to different physical and chemical properties and degradability of the obtained polyurethane material, even if the same raw materials are used, in the reaction process, if an amount of the monomer or the chain extender and reaction conditions are different, a specific chemical structure and degradation performances of the polyurethane obtained will be different, which leads to great differences in specific applications of the polyurethane materials. We found that the degradation time of the polyurethane obtained by using different proportions of monomers and the same process conditions is also different, which leads to different degradation

performances of the materials on the prepared products.

Due to the complexity and diversity of biopolymer materials, the structure and feeding amount of monomers with different compositions and proportions are different, and the chemical structures, degradability and physical and chemical properties of the generated degradable polyurethanes are also quite different. Strictly speaking, even if the feeding monomers are completely the same, the chemical structures of the final products obtained by different feeding amounts and reaction conditions are completely different. Therefore, those skilled in the art have to pay a lot of work to get materials that meet the product characteristics, and can finally get the materials with proper performances through repeated experiments and tests, instead of imagining that the materials can be obtained by adjusting the proportions. Especially for the degradation time of the degradation materials, it is also necessary to carry out a lot of investment to get the optimal data. Therefore, from this point of view, the disclosure is creative.

Although the degradable medical polyurethane and its application in medicine have been reported in many literatures, due to the diversity of materials, there are various solutions for preparing the materials. For example, CN1950098A (Federal Scientific and Industrial Research Organization) disclosed a biodegradable polyurethane degradable vessel supporter in example in the specification of CN1950098A on April 18, 2001, but the document did not give detailed technical parameters, so it was difficult to evaluate the specific physical properties of the product. For example, CN101636187A (Hemoteq AG) discloses a medical polyurethane. A soft segment of the polyurethane adopts a polyol with a molecular weight of 200 to 1,000, which is basically hydrophobic. A molecular weight of a polymer diol in the disclosure is much higher than 1,000, and a chemical structure thereof is completely different. Due to the introduction of a hydrophilic group PEG (200 to 1,000) in a soft segment, a behavior of an obtained polymer in a degradation process is also quite different. For example, CN104744661A was written by us before the patent was formed. Because BDO was used as a chain extender, a synthesized polyurethane product produced slight cytotoxicity. Therefore, we made further research, improvement and optimization in the existing technical solutions, and finally gave up the solution of using the BDO as the chain extender.

At present, after years of research, we have successfully completed batch production of degradable polycaprolactone polyurethane materials with the PEG (200 to 1,000) as the initiator and LDI as a hard segment, which makes it possible for the degradable polycaprolactone polyurethane to be used in product transformation.

The degradable polycaprolactone polyurethane with the LDI as the hard segment has more advantages that:

(1) a degradation product is lysine;

(2) the degradation product will not reduce a pH value of nearby tissues, so it will not cause inflammation;

(3) the surface is easy to bind to a biochemical reagent; and

(4) the surface is friendly to a cell interface, and a non-biological specific effect is small, or the like.

Applying the degradable polycaprolactone polyurethane as a coated stent with the PEG (200 to 1,000) as the initiator has not been reported, and related products have not been launched to the market.

## SUMMARY

Controllable gradient degradation of degradable materials is a big problem in this field. For example, because degradation fragments of polylactic acid materials are uncontrollable, degradation product fragments are too large, which makes a stent tube in the disclosure unable to be used. For example, a degradable ureteral stent in the embodiment of the disclosure does not need secondary extubation in clinic, which greatly reduces risks and pains of patients in secondary operation, and has positive medical effect in clinic. Clinicians are eager to have such products launched on the market, and a large number of research data has also reported that scholars have developed degradable ureteral stents, but none of these degradable ureteral stents have met the requirements of clinical use. Therefore, there are no similar products on the market all over the world, and the development difficulty of medical polymer materials with physical properties, biodegradability and biosecurity that can meet clinical use is obvious.

In order to finely adjust the degradation fragments of the degradable stent, so as to achieve a better clinical effect, the spiral coated stent with controllable gradient degradation of the disclosure is cooperatively realized by a degradation behavior of medical polyurethane materials with controllable gradient degradation prepared by different processes and a gradient degradation characteristic of a magnesium alloy after gradient surface treatment.

The disclosure discloses a spiral coated stent with controllable gradient degradation, including a degradable medical polyurethane and a degradable magnesium alloy material, wherein a soft segment of the degradable medical polyurethane contains a following chemical structure:

PCL-PEG-PCL, wherein a molecular weight of the PEG is 200 to 1,000, and a molecular weight of the PCL is 200 to 10,000;

the degradable magnesium alloy material is of a spiral structure; and

physical properties of the spiral coated stent with controllable gradient degradation need to satisfy the following technical parameters that:
a breaking strength needs to be no less than 1 N, a pressure resistance needs to be no less than 2 N, and a degradation characteristic of the magnesium alloy after surface treatment shows gradient degradation with different time after soaking in an aqueous solution.

The spiral coated stent with controllable gradient degradation according to the disclosure includes the degradable medical polyurethane and the degradable magnesium alloy material, wherein a hard segment of the degradable medical polyurethane is L-lysine diisocyanate, and the soft segment contains the following chemical structure:

PCL-PEG-PCL, wherein the molecular weight of the PEG is 200 to 1,000, and the molecular weight of the PCL is 200 to 5,000;

the degradable magnesium alloy material is of a spiral structure; and

the physical properties of the spiral coated stent with controllable gradient degradation need to satisfy the following technical parameters that:
the breaking strength needs to be no less than 1 N, the pressure resistance needs to be no less than 2 N, and the degradation characteristic of the magnesium alloy after surface treatment shows gradient degradation with different time after soaking in the aqueous solution.

A weight percentage of the degradable medical polyurethane to the degradable magnesium alloy material is 10% to 99%: 1% to 90%.
The spiral coated stent with controllable gradient degradation according to the disclosure includes the degradable medical polyurethane and the degradable magnesium alloy material, wherein the hard segment of the degradable medical polyurethane is L-lysine diisocyanate, and the soft segment contains the following chemical structure:

PCL-PEG-PCL, wherein the molecular weight of the PEG is 200 to 600, and the molecular weight of the PCL is 300 to 3,500; and

the degradable magnesium alloy material is of a spiral structure, which can be woven in a single-strand spiral or multi-strand spiral manner; and

the physical properties of the spiral coated stent with controllable gradient degradation need to satisfy the following technical parameters that:
the breaking strength needs to be no less than 1 N, an elongation at break needs to be no less than 50%, and the pressure resistance needs to be no less than 2 N.

The weight percentage of the degradable medical polyurethane to the degradable magnesium alloy material is 70% to 99%: 1% to 30%.
A chain extender is selected from one of propylene glycol (more preferably 1,3-propanediol) and diamine or diaminelike, and a weight percentage of the chain extender in the degradable medical polyurethane is 1.0% to 20%.
The diamine or diaminelike specifically includes amino acid diaminelike serving as the chain extender, with a structural formula as follows:

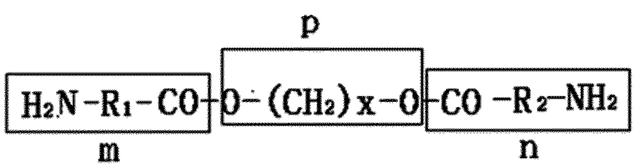
,

wherein, P is a diol, X is a number from 2 to 20, and m and n are one of 20 amino acids commonly used, wherein m

and n can be the same or different.

The diamine includes an amino acid with two amino groups, a derivative and a salt thereof, and specifically includes an amino acid hydrochloride, such as one of lysine methyl ester dihydrochloride, lysine ethyl ester dihydrochloride, cystine methyl ester dihydrochloride, cystine ethyl ester dihydrochloride, ornithine methyl ester dihydrochloride, and ornithine ethyl ester dihydrochloride.

The diol is specifically selected from one or two of glycol, diglycol, tetraglycol, 1,3-propanediol, 1,4-butanediol, 1,5-pentanediol, 1,6-hexanediol, 1,7-heptanediol, 1,8-octanediol, 1,9-nonanediol, and 1,10-decanediol;
for example: two molecular weights of glycine and one molecular weight of 1,3-propanediol are esterified to obtain a compound with two active amino groups connected by two ester bonds, which is specifically as follows:

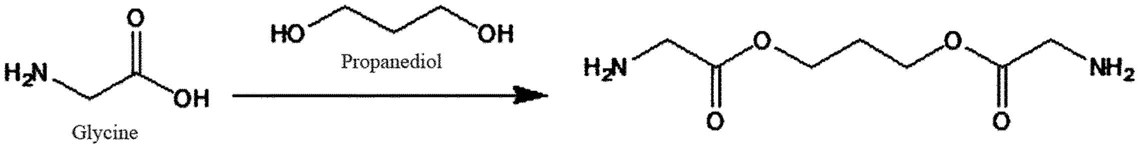


Similar examples are as follows: two molecular weights of alanine and one molecular weight of 1,3-propanediol are esterified to obtain a compound with two active amino groups connected by two ester bonds, one molecular weight of alanine, one molecular weight of valine, and one molecular weight of 1,3-propanediol are esterified to obtain a compound with two active amino groups connected by two ester bond, two molecular weights of leucine and one molecular weight of 1,3-propanediol are esterified to obtain a compound with two active amino groups connected by two ester bonds, isoleucine, leucine and one molecular weight of 1,3-propanediol are esterified to obtain a compound with two active amino groups connected by two ester bonds, two molecular weights of phenylalanine and one molecular weight of 1,3-propanediol are esterified to obtain a compound with two active amino groups connected by two ester bonds, two molecular weights of proline and one molecular weight of 1,3-propanediol are esterified to obtain a compound with two active amino groups connected by two ester bonds, two molecular weights of tryptophan and one molecular weight of 1,3-propanediol are esterified to obtain a compound with two active amino groups connected by two ester bonds, two molecular weights of serine and one molecular weight of 1,3-propanediol are esterified to obtain a compound with two active amino groups connected by two ester bonds, two molecular weights of cysteine and one molecular weight of 1,3-propanediol are esterified to obtain a compound with two active amino groups connected by two ester bonds, two molecular weights of methionine and one molecular weight of 1,3-propanediol are esterified to obtain a compound with two active amino groups connected by two ester bonds, two molecular weights of asparagine and one molecular weight of 1,3-propanediol are esterified to obtain a compound with two active amino groups connected by two ester bonds, two molecular weights of glutamine and one molecular weight of 1,3-propanediol are esterified to obtain a compound with two active amino groups connected by two ester bonds, two molecular weights of threonine and one molecular weight of 1,3-propanediol are esterified to obtain a compound with two active amino groups connected by two ester bonds, and one molecular weight of aspartic acid, one molecular weight of glutamic acid, and one molecular weight of 1,3-propanediol are esterified to obtain a compound with two active amino groups connected by two ester bond, including the compound with two active amino groups connected by two ester bonds obtained by esterifying the combination of various amino acids with one molecular weight of 1,3-propanediol.

A preparation method of a gradient degradable magnesium wire according to the disclosure is as follows: completely soaking a one-meter-long magnesium wire (0.2 mm to 0.5 mm in width, and 0.15 mm in thickness) in a 10%-30% hydrofluoric acid solution or other passivation solution with good biological safety (such as phosphate buffered salt-potassium permanganate solution and phosphate buffered salt-phytic acid solution in a certain proportion disclosed in literature), and lifting by a certain distance every 0.4 minute to 5 minutes to form a gradient passivation protective film with different degradation time, cleaning with acetone after passivation, and then blow-drying for later use.

The degradable medical polyurethane material in the spiral coated stent with controllable gradient degradation according to the disclosure may further include the soft segment which is a polymer diol obtained by copolymerization of one or two of LA, GA, CL, PDO and adipic anhydride with a micromolecule diol (one ofPEG200,PEG300, PEG400, PEG600, glycol, diglycol, 1,3-propanediol, 1,4-butanediol, 1,6-hexanediol, 1,8-octanediol, 1,9-nonanediol, and 1,10-decanediol) as an initiator, and the chain extender is selected from micromolecule diol, diamine or diaminelike, and specifically selected from one or two of glycol, diglycol, tetraglycol, 1,3-propanediol, 1,4-butanediol, 1,6-hexanediol, 1,7-heptanediol, 1,8-octanediol, 1,9-nonanediol, 1,10-decanediol, ethylenediamine, propylene diamine, butanediamine, pentanediamine and amino acid diamine (for example, two segments of compounds with amino groups that are obtained by respectively esterifying carboxyl groups in two molecular weights of amino acid with micromolecule diol); and the degradable polyurethane is prepared by one of the several preparation methods as follows:

first method: using CL with different proportions and PEG with a molecular weight of 200 to 600 to synthesize a

linear polycaprolactone diol, reacting the product with L-lysine diisocyanate, using different diols or diamines as chain extenders and using organic tin or organic bismuth as a catalyst, and reacting to obtain the medical polyurethane adopted in the disclosure;

second method: using PDO and different diols to synthesize a linear PPDO poly diol, reacting the product with different diisocyanates, using different diols or diamines as chain extenders and using organic tin or organic bismuth as a catalyst, and reacting to obtain the medical polyurethane, which can be further added as a stent coating material in order to improve a degradation performance of the stent;

third method: using LA, GA polymer diols, adipic acid polyester diol and oxalic acid polyester diol with different molecular weights initiated by micromolecule diols (one of PEG200-600, glycol, diglycol, 1,3-propanediol, 1,4-butanediol, 1,6-hexanediol, 1,7-heptanediol, 1,8-octanediol, 1,9-nonanediol and 1,10-decanediol) as soft chains, and reacting the same with LDI and different micromolecule diols or diamines, using organic tin or organic bismuth as a catalyst, and reacting to obtain the medical polyurethane, which can be further added as a stent coating material in order to improve the degradation performance of the stent; and

fourth method: using hydroxyl-terminated polydimethylsiloxane as a soft segment and reacting with LDI and micromolecule diol or diamine, and using organic tin or organic bismuth as a catalyst to form an organosilicon-polyurethane block copolymer composed of softs segment and hard segments alternately, which can be further added as a stent coating material in order to improve the degradation performance of the stent.

The organic tin and the organic bismuth are selected from commercially available medical catalysts, such as bismuth octoate, bismuth laurate, bismuth neodecanoate, bismuth naphthenate, bismuth oxide, and the like, and preferably stannous octoate or bismuth octanoate.

The spiral coated stent with controllable gradient degradation according to the disclosure may further include other ydrophobic polymer materials and hydrophilic polymer materials, wherein the hydrophobic polymer materials include: polylactic acid, polycaprolactone, poly(p-dioxanone) and copolymers thereof(PPDO, PLA-PDO) poly(p-dioxanone) (PPDO), polytrimethylene carbonate, polylactic acid-trimethylene carbonate copolymer, polycaprolactone-trimethylene carbonate copolymer, polyglycolic acid, polylactic acid-glycolic acid copolymer, polyether-ether-ketone, polyvinylpyrrolidone and/or polyethylene glycol, polyvalerolactone, poly-ε-decanolactone, polylactide, polyglycolide, a copolymer of polylactide and polyglycolide, poly-ε-caprolactone, polyhydroxybutyric acid, polyhydroxybutyrate, polyhydroxyvalerate, polyhydroxybutyrate-co-valerate, poly(1,4-dioxane-2,3-dione), poly(1,3-dioxan-2-one), poly(p-dioxanone), polyanhydride (such as polymaleic anhydride), polyhydroxymethacrylate, polycyanoacrylate, polycaprolactone dimethacrylate, poly-β-maleic acid, polycaprolactone butyl acrylate, multi-block polymers from oligocaprolactone diol and oligodioxanone diol, polyethylene glycol and polybutylene terephthalate)), polyvalerolactone, polyglycolic acid trimethyl carbonate, polycaprolactone-glycolide, poly(y-ethylglutamate), poly(DTH-iminocarbonate), poly(DTE-co-DT-carbonate), poly(bisphenol A-iminocarbonate), polyorthoester, poly(trimethylglycolate), poly(trimethylcarbonate), polyurethane carbonate, poly(N-vinyl)-pyrrolidone, polyvinyl alcohol, polyesteramide, alcoholized polyester, polyphosphate, polyphosphazene, poly[p-carboxyphenoxy)propane], polyhydroxyvaleric acid, polyanhydride, polyoxyethylene-propylene oxide, soft polyurethane, polyurethane having amino acid residues in a main chain, polyether ester (such as polyethylene oxide), polyolefin oxalate, polyorthoester and copolymers thereof, and preferably one of polylactic acid, polycaprolactone, poly(p-dioxanone) and copolymers thereof (PPDO, PLA-PDO) poly(p-dioxanone) (PPDO), polytrimethylene carbonate, polylactic acid-trimethylene carbonate copolymer, polycaprolactone-trimethylene carbonate copolymer, polyglycolic acid, and polylactic acid-glycolic acid copolymer; and a viscosity-average molecular weight of the biodegradable polymer material is 500 to 1,000,000, which is used for adjusting one of a softness and a hardness of the material; and
the hydrophilic polymer materials include one of alginate, modified alginate and alginate degraded into hexosamine and N-acetylglucosamine, polyvinyl pyrrolidone series, starch, starch grafted acrylonitrile, starch grafted hydrophilic monomer, carrageenin, fibrinogen, collagen, protein-containing polymer, polyamino acid, synthetic polyamino acid, corn protein, polyacrylate, vinyl acetate copolymer, modified polyvinyl alcohols, carboxymethyl cellulose, cellulose grafted acrylonitrile, cellulose grafted acrylate, cellulose xanthate grafted acrylate, cellulose grafted acrylamide, and epichlorohydrin cross-linked carboxymethyl cellulose; or one of macromolecular antibacterial absorbent materials, polyamino acid, chitosan, polylysine, and polyvinyl alcohol.

The degradable magnesium alloy material in the spiral coated stent with controllable gradient degradation according to the disclosure is selected from materials refined from various chemical elements harmless to a human body, specifically including one or a combination of two of high-purity magnesium (purity greater than 99.0%), magnesium-iron alloy (weight percentage of 1: 0.01 to 1), magnesium-zinc alloy (weight percentage of 1: 0.01 to 1), magnesium-calcium alloy (weight percentage of 1: 0.01 to 1), and magnesium-aluminum alloy (weight percentage of 1: 0.01 to 1),

preferably high-purity magnesium and magnesium-zinc alloy (weight percentage of 1: 0.01 to 1 preferably) such as Mg-Nd-Zn-Zr, Mg-Zn-Mn, Mg-Zn-Zr, Mg-Zn-Mn-Se-Cu, and Mg-Zn binary alloy.

A first preparation method of the spiral coated stent with controllable gradient degradation according to the disclosure is as follows:

(1) preparing a gradient degradable magnesium wire: completely soaking a round wire or a flat wire of a one-meter-long magnesium wire (0.1 mm to 2 mm in width, and 0.1 mm to 0.5 mm in thickness) in a dipotassium hydrogen phosphate aqueous solution containing phytic acid with a certain concentration or a 5%-30% hydrofluoric acid solution, and lifting by 1 cm to 10 cm every 1 minute to 10 minutes to form a gradient passivation protective film, marking one end passivated for short time as B end and marking one end passivated for long time as A end;

(2) crimping the degradable magnesium alloy wire processed in step (1) into a spiral pattern (single-strand spiral or reverse multi-strand spiral) (shown in FIGs. 1 and 2), dissolving a degradable medical polyurethane material or a composite material in an organic solvent (one or two selected from heptane, decane, tetrahydrofuran, isoamyl acetate, hexane, dichloromethane, chloroform, cyclohexanone and dimethylformamide) to prepare a coating material (material concentration of 5% to 50%), and evenly spraying the coating material on a surface of a stent through an electrostatic spinning nozzle in the continuous rotation process of the step (1) to manufacture the same into a coated composite stent with a thickness of 0.001 mm to 1 mm, and preferably 0.01 mm to 0.5 mm; and

(3) dissolving a hydrophilic material in water to prepare a required concentration, and dip-coating or evenly spraying on the surface of the stent to manufacture the same into a water-soluble coating, which is convenient for clinicians to place and use.

A second preparation method of the spiral coated stent with controllable gradient degradation according to the disclosure is as follows:

(1) preparing a gradient degradable magnesium wire: completely soaking a round wire or a flat wire of a one-meter-long magnesium wire (0.1 mm to 2 mm in width, and 0.1 mm to 0.5 mm in thickness) in a dipotassium hydrogen phosphate aqueous solution containing phytic acid with a certain concentration or a 5%-30% hydrofluoric acid solution, and lifting by 1 cm to 10 cm every 1 minute to 10 minutes to form a gradient passivation protective film, marking one end passivated for short time as B end and marking one end passivated for long time as A end;

(2) crimping the degradable magnesium alloy wire processed in step (1) into a spiral pattern (single-strand spiral or reverse multi-strand spiral) (shown in FIGs. 1 and 2), threading with tetrafluoroethylene or metal rod, spirally fixing the magnesium wire in a special grinding tool for manufacturing corrugated pipes according to a processing technology for manufacturing corrugated pipes, extruding the degradable medical polyurethane material with a tubule extruder to manufacture the same into a coated composite stent with a thickness of 0.001 mm to 1 mm, and preferably 0.01 mm to 0.5 mm; and

(3) dissolving a hydrophilic material in water to prepare a required concentration, and dip-coating or evenly spraying on the surface of the stent to manufacture the same into a water-soluble coating, which is convenient for clinicians to place and use.

The coated stent of the disclosure can also be processed as follows:

when preparing a vascular stent, an anticoagulant ingredient is cross-linked and fixed on a surface of a treated bare stent by means of a cross-linking agent such as glutaraldehyde, and a coagulation ingredient may be hirudin, heparan sulfate and derivatives thereof such as complete desulfurization and N-reacetyl heparin desulfurization and N-reacetyl heparin, so that an anticoagulant coating which does not activate blood coagulation is prepared.

a surface of a degradable magnesium alloy is passivated to form a corrosion-resistant nontoxic conversion film via commonly used methods like a phosphate conversion film method, a phytic acid conversion film method, a rare earth salt conversion film method, and an organic conversion film method, or fluorination treatment is carried out on the surface of the bare stent, and the specific method includes:
polishing an untreated biodegradable magnesium alloy stent, completely soaking the stent in 5-25g/L dipotassium hydrogen phosphate aqueous solution containing 0.5-1.5g/L phytic acid at a temperature of 40°C to 70°C, and lifting for a certain distance at intervals according to the need of degradation time to form a gradient passivation protective film.

The untreated biodegradable magnesium alloy stent is polished, completely soaked in a hydrofluoric acid with a mass percentage of 10% to 40% for 10 minutes to 120 minutes, and lifted for a certain distance at intervals according to the need of degradation time to form the gradient passivation protective film.

According to the spiral coated stent with controllable gradient degradation of the disclosure, commercially available or published polypeptides, proteins and active ingredients, including anti-proliferation, anti-migration, anti-angiogenesis, anti-inflammation, antiphlogosis, cell growth inhibition, cytotoxicity or anti-thrombosis drugs with physiological activity, can be added into the polyurethane materials according to the needs of clinical treatment, fior example, including: sirolimus, everolimus, pimecrolimus, melphalan, ifosfamide, trofosamine, chlorambucil, bendamustine, somatostatin, tacrolimus, roxithromycin, daunomycin, ascomycin, baflomycin, lomustine, cyclophosphamide, estramustine, dacarbazine, erythromycin ethylsuccinate, midecamycin, josamycin, canavalin, clarithromycin, troleandomycin, catharanthine, vincristine, vindesine, vinorelbine, etoposide, teniposide, nimustine, carmustine, busulfan, procarbazine, treosulfan, temozolomide, thiotepa, doxorubicin, aclarubicin, epirubicin, mitoxantrone, idarubicin, bleomycin, mitomycin C, dactinomycin, methotrexate, fludarabine, fludarabine-5'-dihydrophosphate, cladribine, mercaptopurine, thioguanine, cytarabine, fluorouracil, gemcitabine, capecitabine, docetaxel, carboplatin, cisplatin, oxaliplatin, rosuvastatin, atorvastatin, pravastatin, pitavastatin, polyoxin, cerivastatin, simvastatin, lovastatin, fluvastatin, amsacrine, irinotecan, topotecan, hydroxyurea, miltefosine, pentostatin, aldesleukin, tretinoin, asparaginase, oncaspar, anastrozole, exemestane, letrozole, formestane, aminoglutethimide, bromerguride, bromocriptine, elymoclavine, ergocristine, ergocryptine, ergodine, ergotoxine, ergosine, ergosinine, ergobasinine, ergotamine, ergotamine, ergotaminine, ergovaline, ergocorninine, ergocryptine, ergocryptinine, ergometrine, lergotrile, lisuride, ergosterol, lysergic acid, adriamycin, azithromycin, spiramycin, cepharanthine, 8-$\alpha$-ergoline, dimethyl ergoline, agroclavine, 1-allyl lisuride, 1-allyl terguride, lysergic acid amide, lysergic acid diethylamine, isolysergic acid, isoergotamide, isolysergic acid diethylamine, mesulergine, thalidomide, (5-isoquinoline sulfonyl) homopiperazine, cyclosporine, smooth muscle cell proliferation inhibitor 2$\omega$, metergoline, methylergonovine, dimethylergonovine, pergolide, proterguride and terguride, celecoxib, epothilone A and B, mitoxantrone, azathioprine, mycophenolate mofetil, antisense c-myc, antisense b-myc, betulinic acid, camptothecin, mupaforst, melanocyte stimulating hormone, active protein C, interleukin 1-$\beta$-inhibitor, $\beta$-lapaqinone, podophyllotoxin, betulin, 2-ethyl hydrazine of podophyllic acid, molgramostim, polyethylene glycol interferon $\alpha$-2b, lenograstim, filgrastim, dacarbazine, baliximab, daclizumab, selectin, cholesterol ester transporter inhibitor, cadherin, cytokine inhibitor, cyclooxygenase-2 inhibitor, thymosin $\alpha$-1, fumaric acid and esters thereof, calcipotriol, tacalcitol, lapasol, nuclear factor kB, angiotensin, ciprofloxacin, fluoxetine, monoclonal antibody for inhibiting muscle cell proliferation, bovine basic fibroblast growth factor antagonist, probucol, prostaglandin, 1,11-dimethoxycoumarin-6-one, 1-hydroxy-11-methoxycoumarin-6-one, scopoletin, colchicine, nitric oxide donor, tamoxifen, diethylstilbestrol, medroxyprogesterone acetate, estradiol valerate, estradiol benzoate, tranilast, verapamil, tetrandrine, $\beta$-estradiol, $\alpha$-estradiol, estriol, estrone, ethinyl estradiol, tyrosine kinase inhibitor, cyclosporine A, taxol and derivatives thereof, synthesized and natural tricarbon dioxide macrocyclic oligomer and derivatives thereof, monobenzoxazone, acetaminophen, diclofenac, lonazolac, dapsone, o-carbamoylphenoxy-acetic acid, lidocaine, ketoprofen, mefenamic acid, tumstatin, avastin, hydroxychloroquine, auranofin, sodium aurothiosuccinate, oxaceprol, celecoxib, $\beta$-sitosterol, ademetionine, myrtecaine, polidocanol, vanillylnonanamide, levomenthol, benzocaine, aescin, allitinib, demecolcine, cytochalasin A-E, indanocine, nocodazole, piroxicam, meloxicam, chloroquine phosphate, penicillamine, S100 protein, bacitracin, vitronectin receptor antagonist, azelastine, guanidine cyclase stimulator, tissue inhibitor of metalloproteinase 1 and 2, free nucleic acid, nucleic acid incorporated with viral transmitter, deoxyribonucleic acid and ribonucleic acid fragment, plasminogen activator inhibitor 1, plasminogen activator inhibitor 2, antisense oligonucleotide, vascular endothelial growth factor inhibitor, insulin-like growth factor 1, active agent from antibiotic group, penicillins, antithrombotic agents, desulfurized and N-acetylated heparin, tissue plasminogen activator, GpIIb/IIIa platelet membrane receptor, factor Xa inhibitor antibody, heparin, hirudin, r-hirudin, D-phenylalanyl-L-prolyl-L-arginine chloromethyl keton, acetylcholinesterase inhibitor, thioprotease inhibitor, prostacyclin, vapiprost, interferons $\alpha$, $\beta$ and $\gamma$, histamine antagonist, serotonin blocker, apoptosis inhibitor, apoptosis regulator, protamine, sodium salt of 2-methylthiazolidine-2,4-dioctyl phthalate, prourokinase, streptokinase, warfarin and urokinase, vasodilator agent, platelet-derived growth factor antagonism, chloropiperaquinone bromide, nifedipine, tocopherol, vitamins B1, B2, B6 and B12, folic acid, molsidomine, tea polyphenol, epicatechin gallate, epigallocatechin gallate, leflunomide, anakinra, etanercept, procainamide, retinoic acid, quinidine, disopyramide, flecainide, propafenone, sotalol, amiodarone, natural or synthetic steroid, inonotus obliquus alcohol, maguire glycoside A, sulfasalazine, etoposide, triamcinolone, sisomicin, metapleigenin glycoside, mansonine, streblus glycoside, episisomicin, richoloma velutin, mantonen, streblus glycoside, hydrocortisone acetate, betamethasone, dexamethasone, nonsteroidal antiinflammatory substance, antifungal agent, anti-protozoan agent, natural terpenoid, 4,7-oxo-saposhnikovia divaricate oxalic acid, dryland chrysanthemum terpenes B1, B2, B3 and B7, tubeimoside, anti-dysentery bruceols A, B and C, anti-dysentery bruceoside C, brucaldine javanicas N and P, iso-deoxyelephantopin, elephantopus tomentosus lactones A and B, dihydroxypropyltheo-pyllines A, B, C and D, and amethystoidins A and B, nitidine chloride, 12-$\beta$-hydroxypregnadiene-3,20-diketone, longikaurin B, sculponeatin C, isodon excisoide terpenes A and B, rabdosia racemosa terpenes A and B, taxinines A and B, raloxinol, triptolide, ursolic acid, jatamasic acid A, isoiridogermanal, maytenfoliol, cymarin, strophanthin, aristolochic acid, aminopterin, hydroxylamine pterin, anemonin, protoanemonin, berberine, chelidonin chloride, cicutoxin, tribobine, cudrania isoflavone A, curcuma longa, dihydronitidine, ginkgol, ginkgol, ginkgolic acid, and the like, and salts, hydrates, solvates, enantiomers, racemic compounds of the above active agents.

The spiral coated stent with controllable gradient degradation according to the disclosure may be used for preparing various tract stents in vivo, specifically including: blood vessel, vein, gullet, biliary tract, trachea, bronchus, small intestine, large intestine, urethra, ureter or other ducts similar to a tubular passage, and specifically including vascular stents, tracheal stents, bronchial stents, urethral stents, gullet stents, biliary stents, ureteral stents (double J tubes), ureteral stenosis stents, stents for small intestine, stents for large intestine, laryngeal implants, bypass catheters or ileostomy.

One or two contrast mediums, for example, common contrast agents such as zirconium dioxide, barium sulfate, and iodine preparations, may also be added in the spiral coated stent with controllable gradient degradation according to the disclosure, which may be used for in vivo development.

BRIEF DESCRIPTION OF THE DRAWINGS

FIG. 1 is a schematic diagram of a single-strand spiral magnesium alloy stent;

FIG. 2 is a schematic diagram of a double-strand spiral magnesium alloy stent;

FIG. 3 is a schematic diagram of a multi-strand spiral magnesium alloy stent;

FIG. 4 is a schematic diagram of a degradable ureteral stent; and

FIG. 5 is a partial enlarged schematic diagram of the degradable ureteral stent.

**DETAILED DESCRIPTION**

Examples of magnesium alloy constituents selected in Embodiments 1 to 9 are as follows:

| | Zn(wt%) | Mn(wt%) | Se(wt%) | Sr(wt%) | Cu(wt%) | Ca(wt%) | Zr | Mg(wt%) |
|---|---|---|---|---|---|---|---|---|
| 1 | 0.2 | 0.5 | | | | | | Balance |
| 2 | 4.0 | 0.9 | 0.1 | | | | 0.5 | Balance |
| 3 | | 1.2 | | | | | | Balance |
| 4 | 0.2 | | 1.5 | | 0.2 | | | Balance |
| 5 | | | 0.1 | 0.1 | | 0.1 | | Balance |
| 6 | 1.2 | | 0.2 | | | | | Balance |
| 7 | 5.0 | | | - | 0.1 | 0.2 | 0.1 | Balance |
| 8 | 4.0 | 0.2 | | 0.1 | | | | Balance |
| 9 | 4.2 | 0.1 | | 0.2 | - | | 0.1 | Balance |

Embodiment 1: Tracheal coated stent

A preparation process of the tracheal coated stent was as follows:

(1) weaving a degradable metal material (alloying constituent proportions were selected according to Table 2) wire into double-strand or multi-strand spiral stents (shapes as shown in FIG. 2 and FIG. 3), or cutting a magnesium alloy tube into a pattern or a porous shape of a commercially available vascular stent with spiral structure (the stent had a diameter of 6.5 mm, a length of 4 cm, and a wall thickness of 0.2 mm);

(2) polishing the stent prepared in step (1), soaking the stent in hydrofluoric acid with a mass percentage of 20% for 10 minutes, taking the stent out, and then washing with acetone, and drying;

(3) dissolving a degradable medical polyurethane material (polyurethane with PEG300 as an initiator, PCL diol as a soft segment and L-lysine diisocyanate as a hard segment, a number average molecular weight of 150,000 to 170,000, a breaking strength of 35 MPa, and an elongation at break of 350%) in a chloroform solvent to prepare a 8%-15% solution; and

(4) dip-coating or evenly spraying the solution in (3) on the stent in (2) to manufacture the same into the coated stent (the stent had a tube wall thickness of 0.26 mm).

Embodiment 2: Drug-loading trachea coated stent

A preparation process of the drug-loading trachea coated stent was as follows:

(1) weaving a degradable material (alloying constituent proportions were selected according to Table 2) wire into double-strand or multi-strand spiral stents (shapes as shown in FIG. 2 and FIG. 3), or cutting a magnesium alloy tube into a pattern or a porous shape of a commercially available tracheal stent with spiral structure (the stent had a diameter of 7 mm, a length of 6 cm, and a wall thickness of 0.2 mm);

(2) polishing the stent prepared in step (1), soaking the stent in hydrofluoric acid with a mass percentage of 20% for 12 minutes, taking the stent out, and then washing with anhydrous acetone, and drying;

(3) dissolving a degradable medical polyurethane material (polyurethane with PEG300 as an initiator, PCL diol as a soft segment and L-lysine diisocyanate as a hard segment, a number average molecular weight of 190,000 to 210,000, a breaking strength of 35 MPa, and an elongation at break of 350%) in a chloroform solvent to prepare a 8%-15% solution, and dissolving drugs (such as paclitaxel with a dosage range of 0.5 $ug/mm^2$ to 10 $ug/mm^2$) needed to treat malignant tumors or prevent restenosis in the prepared polyurethane chloroform solution;

(4) dip-coating or evenly spraying the solution in (3) on the stent in (2) to manufacture the same into the coated stent (the stent had a tube wall thickness of 0.28 mm); and;

(5) dip-coating or spraying the composite material prepared in (4) with a hydrophilic coating (such as aqueous solution made of chitosan, hyaluronic acid, collagen, cellulose, etc.), blowing-drying, polishing and grinding to manufacture the same into the coated stent.

Embodiment 3: Urethral coated stent

A preparation process of the urethral coated stent was as follows:

(1) weaving a degradable metallic material (alloying constituent proportions were selected according to Table 2) wire into double-strand or multi-strand spiral stents (shapes as shown in FIG. 2 and FIG. 3), or cutting a magnesium alloy tube into a pattern or a porous shape of a commercially available vascular stent with spiral structure (the stent had a diameter of 4.0 mm, a length of 2.5 cm, and a wall thickness of 0.16 mm);

(2) polishing the stent prepared in (1), soaking the stent in hydrofluoric acid with a mass percentage of 10% for 20 minutes to 30 minutes, taking the stent out, and then washing with acetone, and drying;

(3) dissolving a degradable medical polyurethane material (polyurethane with PEG600 as an initiator, PCL diol as a soft segment and L-lysine diisocyanate as a hard segment, a number average molecular weight of 60,000 to 80,000, a breaking strength of 35 MPa, and an elongation at break of 250%) in a chloroform solvent to prepare a 8%-15% solution for later use;

(4) dip-coating or evenly spraying the solution in (3) on the stent in (2) to manufacture the same into the coated stent (the stent had a tube wall thickness of 0.2 mm to 0.23 mm); and

(5) dip-coating or spraying the composite material prepared in (4) with a hydrophilic coating (such as aqueous solution made of chitosan, hyaluronic acid, collagen, cellulose, etc.), blowing-drying, polishing and grinding to manufacture the same into the coated stent.

Embodiment 4: In vitro degradation experiments of the three stents according to the embodiments

The stents obtained in the above embodiments were soaked in a PBS solution, the solution was changed once a week, and degradation of the stents was observed for a long time at 37°C. The results were as follows:

| Embodiment | Before soaking | | 15 days | | 1 month | | 2 months | | 3 months | | 6 months | |
|---|---|---|---|---|---|---|---|---|---|---|---|---|
| | Breaking strength | Compressive strength | Breaking strength | Compressive strength | Breaking strength | Compressive strength | Breaking strength | Compressive strength | Breaking strength | Compressive strength | Breaking strength | Compressive strength |
| 1 | 3.5N | 6.0N | 3.5N | 6.0N | 3.0N | 5.2N | 2.5N | 4.0N | 2.0N | 3.0N | 1.5N | 2.0N |
| 2 | 3.3N | 5.2N | 3.1N | 5.1N | 3.0N | 5.0N | 3.0N | 4.2N | 2.5N | 3.5N | 1.9N | 2.5N |
| 3 | 3.0N | 3.6N | 2.8N | 4.0N | 2.5N | 1.3N | - | - | - | - | - | - |

Embodiment 5: First preparation method of ureteral stent with gradient degradation (degradation time lasted for 1 to 2 months)

1. Preparation of a gradient degradable magnesium wire

A one-meter-long magnesium wire (0.2 mm to 0.3 mm in width, and 0.12 mm in thickness) was completely soaked in a 15 g/L dipotassium hydrogen phosphate aqueous solution containing 1.2 g/L phytic acid at about 60°C, and lifted by 5 cm every 2.5 minutes to form a gradient passivation protective film, one end passivated for short time was marked as B end and one end passivated for long time was marked as A end.

2. Preparation of a degradable ureteral stent

(1) The gradient degradable magnesium alloy wire (alloying constituent proportions were selected according to Table 9) processed was crimped into a single-strand spiral shape, with a pitch of 1.8 mm to 2.5 mm (as shown in FIG. 1) and a total stent length of 26 cm to 28 cm;

(2) a degradable medical polyurethane material (polyurethane with PEG400 as an initiator, PCL as a soft segment and L-lysine diisocyanate as a hard segment, a number average molecular weight of 50,000 to 70,000 (referring to Solution 9 in Embodiment 10 for specific synthesis method), a breaking strength of 20 MPa, and an elongation at break of 250%) was dissolved in a chloroform solvent to prepare a 8%-15% solution for later use;

(3) the solution in (2) was repeatedly dip-coated or evenly sprayed on the stent in (1) to manufacture the same into the coated stent (the stent had a tube wall thickness of 0.2 mm); (as shown in FIG. 4); and

(4) the composite material prepared in (3) was dip-coated or spraye with a hydrophilic coating (such as aqueous solution made of PVA, PVP, and micromolecule hyaluronic acid, etc.), aired, polished and ground into the coated stent.

3. Degradation experiment

The ureteral stent prepared in this embodiment was soaked in artificial urine, and the artificial urine was changed every other week. After 20 days, it was observed that short flocculent degradation products began to appear on a surface of the stent tube, and then the short flocculent degradation products became more and more; until 30 days, only the spiral magnesium wire remained, and then the magnesium wire gradually broke into fragments less than 2 mm from the B end to the A end; and after 40 days, the B end remained only was also degraded into debris.

Embodiment 6: Second preparation method of ureteral stent with gradient degradation (degradation time lasted for 2 to 3 months)

1. Preparation of a gradient degradable magnesium wire

A one-meter-long magnesium wire (0.2 mm to 0.4 mm in width, and 0.15 mm in thickness) was complete soaked in a 10% hydrofluoric acid solution, and lifted by 4 cm every 0.5 minute to form a gradient passivation protective film, one end passivated for short time was marked as B end and one end passivated for long time was marked as A end, then washed with acetone after the passivation was ended, and blow-dried for later use.

2. Preparation of a degradable ureteral stent

(1) The gradient degradable magnesium alloy wire (alloying constituent proportions were selected according to Table 8) processed was crimpied into a single-strand spiral shape, with a pitch of 2.0 mm to 2.5 mm (as shown in FIG. 1) and a total stent length of 24 cm to 28 cm;

(2) A degradable medical polyurethane material (polyurethane with PEG200 as an initiator, a copolymer diol of PCL and GA as a soft segment and L-lysine diisocyanate as a hard segment, a number average molecular weight of

120,000 to 150,000 (referring to Solution 5 in Embodiment 10 for specific synthesis method), a breaking strength of 30 MPa, and an elongation at break of 350%) was dissolved in a chloroform solvent to prepare a 8%-15% solution for later use;

(3) the solution in (2) was repeatedly dip-coated or sprayed on the stent in (1) to manufacture the same into the coated stent (the stent had a tube wall thickness of 0.28 mm); (as shown in FIG. 4 and FIG. 5); and

(4) the composite material prepared in (3) was dip-coated or sprayed with a hydrophilic coating (such as aqueous solution made of PVA, PVP, and micromolecule hyaluronic acid, etc.), aired, polished and ground into the coated stent.

3. Degradation experiment

The ureteral stent prepared in this embodiment was soaked in artificial urine, and the artificial urine was changed every other week. After 60 days, it was observed that short flocculent degradation products began to appear on a surface of the stent tube, and then the short flocculent degradation products became more and more; until 70 days, the spiral magnesium wire and trace attachments remained. In the following days, the magnesium wire gradually broke into fragments less than 2 mm from the B end to the A end; and after 90 days, the B end remained only was also degraded into debris.

Embodiment 7: Third preparation method of ureteral stent with gradient degradation (degradation time lasted for 3 to 5 months)

1. Preparation of a gradient degradable magnesium wire

A one-meter-long magnesium wire (0.3 mm to 0.4 mm in width, and 0.15 mm in thickness) was completely soked in a 10% hydrofluoric acid solution, and lifted by 3 cm every 0.4 minute to form a gradient passivation protective film, one end passivated for short time was marked as B end and one end passivated for long time was marked as A end, then washed with acetone after the passivation was ended, and blow-dried for later use.

2. Preparation of a degradable ureteral stent

(1) The gradient degradable magnesium alloy wire (alloying constituent proportions were selected according to Table 8) processed was crimped into a single-strand spiral shape, with a pitch of 2.2 mm to 2.6 mm (as shown in FIG. 1) and a total stent length of 24 cm to 28 cm;

(2) a degradable medical polyurethane material and PLGA (a weight ratio of 2:1) (polyurethane with PEG400 as an initiator, PCL as a soft segment and L-lysine diisocyanate as a hard segment, a number average molecular weight of 80,000 to 100,000 (referring to Solution 3 in Embodiment 10 for specific synthesis method), a breaking strength of 30 MPa, and an elongation at break of 300%), PLGA (LA:GA=8:2, a viscosity-average molecular weight of 60,000 to 80,000) were dissolved in a chloroform solvent to prepare a 8%-15% solution for later use;

(3) the solution in (2) was repeatedly dip-coated or sprayed on the stent in step (1) to manufacture the same into the coated stent (the stent had a tube wall thickness of 0.30 mm); (as shown in FIG. 4); and

(4) the composite material prepared in (3) was dip-coated or sprayed with a hydrophilic coating (such as aqueous solution made of PVA, PVP, and micromolecule hyaluronic acid, etc.), aired, polished and ground into the coated stent.

3. Degradation experiment

The ureteral stent prepared in this embodiment was soaked in artificial urine, and the artificial urine was changed every other week. After 70 days, it was observed that short flocculent degradation products began to appear on a surface of the stent tube, and then the short flocculent degradation products became more and more; until 90 days, the spiral magnesium wire and trace attachments remained. In the following days, the magnesium wire gradually broke into fragments less than 2 mm from the B end to the A end; and after 120 days, the B end remained only was also degraded

into debris.

Embodiment 8: Fourth preparation method of ureteral stent with gradient degradation (degradation time lasted for 20 to 30 days)

1. Preparation of a gradient degradable magnesium wire

A one-meter-long magnesium wire (0.2 mm to 0.4 mm in width, and 0.15 mm in thickness) was completely soaked in a 10% hydrofluoric acid solution, and lifted by 3 cm every 0.4 minute to form a gradient passivation protective film, one end passivated for short time was marked as B end and one end passivated for long time was marked as A end, then washed with acetone after the passivation was ended, and blow-dried for later use.

2. Preparation of a degradable ureteral stent

(1) The gradient degradable magnesium alloy wire (alloying constituent proportions were selected according to Table 8) processed was crimped into a single-strand spiral shape, with a pitch of 2.2 mm to 2.6 mm (as shown in FIG. 1) and a total stent length of 28 cm to 32 cm;

(2) a degradable medical polyurethane material and PLGA (a weight ratio of 5:1) (polyurethane with PEG400 as an initiator, PCL as a soft segment and L-lysine diisocyanate as a hard segment, a number average molecular weight of 50,000 to 70,000 (referring to Solution 1 in Embodiment 10 for specific synthesis method), a breaking strength of 20 MPa, and an elongation at break of 200%), PLGA (LA:GA=6:4, a viscosity-average molecular weight of 40,000 to 60,000) were dissolved in a chloroform solvent and dried into evenly mixed powder, adding the powder into a precise tubule extruder, the magnesium wire in step (1) was spirally fixed in a grinding tool for manufacturing corrugated pipes, the precise tubule extruder was heated to 60°C to 70°C, the material in step (2) was melt, and extruded into the grinding tool to manufacture the same into a coated stent; and

(3) the composite material prepared in (2) was dip-coated or sprayed with a hydrophilic coating (such as aqueous solution made of PVA, PVP, and micromolecule hyaluronic acid, etc.), aired, polished and ground into the coated stent with a hydrophily coating.

3. Degradation experiment

The ureteral stent prepared in this embodiment was soaked in artificial urine, and the artificial urine was changed every other week. From 20 days, it was observed that flocculent degradation products began to appear on a surface of the stent tube, and completely degraded into debris after 30 days.

Embodiment 9: Fourth preparation method of antibacterial drug-carrying ureteral stent with gradient degradation (degradation time lasted for 2 to 3 months)

1. Preparation of a gradient degradable magnesium wire

A one-meter-long magnesium wire (0.2 mm to 0.4 mm in width, and 0.13 mm in thickness) was completely soaked in a 10% hydrofluoric acid solution, and lifted by 7 cm every 1 minute to form a gradient passivation protective film, one end passivated for short time was marked as B end and one end passivated for long time was marked as A end, then washed with acetone after the passivation was ended, and blow-dried for later use.

2. Preparation of a degradable ureteral stent

(1) The gradient degradable magnesium alloy wire (alloying constituent proportions were selected according to Table 8) processed was crimed into a single-strand spiral shape, with a pitch of 1.8 mm to 2.2 mm (as shown in FIG. 1) and a total stent length of 26 cm to 28 cm;

(2) a degradable medical polyurethane material and PLGA (a weight ratio of 2:1) (polyurethane with PEG600 as an

initiator, PCL and GA as soft segments and L-lysine diisocyanate as a hard segment, a number average molecular weight of 100,000 to 120,000 (referring to Solution 4 in Embodiment 10 for specific synthesis method), a breaking strength of 20 MPa, and an elongation at break of 300%), PLGA (LA:GA=6:4, a viscosity-average molecular weight of 50,000 to 60,000) were dissolved in a chloroform solvent, and an appropriate amount of antibacterial drugs (which may be one of drugs such as ionic silver, chlorhexidine, antibacterial peptide, quaternary ammonium salt, antibiotics, etc., which could be used in medical appliances) was added into the solution and mixed evenly to prepare a 8%-15% solution for later use;

(3) the solution in (2) was repeatedly dip-coated or sprayed on the stent in (1) to manufacture the same into the coated stent (the stent had a tube wall thickness of 0.3 mm to 0.35 mm); (as shown in FIG. 4); and

(4) the composite material prepared in (3) was dip-coated or sprayed with a hydrophilic coating (such as aqueous solution made of PVA, PVP, and micromolecule hyaluronic acid, etc.), aired, polished and ground into the coated stent.

Embodiment 10: synthesis solutions of polycaprolactone polyurethane were as follows:

Solution 1:

9.00 g of ε-caprolactone, and 3.00 g of PEG-600 were respectively weighed and added into a test tube with stannous octoate as a catalyst, then a magnetic stirrer was added, vacuumized/filled with nitrogen for three times. Then, an opening of the test tube was sealed under vacuumizing, and the test tube was put into an oil bath pan at 140°C to react for 24 hours to obtain a linear polymer. Then 2 g of L-lysine diisocyanate and 0.55 g of 1,3-propanediol were weighed, added into the test tube, vacuumized and the opening of the test tube was sealed. The test tube was put into an oil bath pan at 70°C to react for 4 hours to obtain the final product (a number average molecular weight was tested to be 50,000 to 70,000 by GPC).

Solution 2:

9.00 g of ε-caprolactone, and 3.00 g of PEG-200 were respectively weighed and added into a test tube with stannous octoate as a catalyst, then a magnetic stirrer was added, vacuumized/filled with nitrogen for three times. Then, an opening of the test tube was sealed under vacuumizing, and the test tube was put into an oil bath pan at 140°C to react for 24 hours to obtain a linear polymer. Then 3 g of L-lysine diisocyanate and 0.73 g of 1,3-propanediol were weighed, added into the test tube, vacuumized and the opening of the test tube was sealed. The test tube was put into an oil bath pan at 70°C to react for 4 hours to obtain the final product (a number average molecular weight was tested to be 60,000 to 80,000 by GPC).

Solution 3:

12.00 g of ε-caprolactone, and 3.00 g of PEG-600 were respectively weighed and added into a test tube with stannous octoate as a catalyst, then a magnetic stirrer was added, vacuumized/filled with nitrogen for three times. Then, an opening of the test tube was sealed under vacuumizing, and the test tube was put into an oil bath pan at 140°C to react for 24 hours to obtain a linear polymer. 3 g of L-lysine diisocyanate and 0.76 g of 1,3-propanediol were weighed, added into the test tube, vacuumized and the opening of the test tube was sealed. The test tube was put into an oil bath pan at 70°C to react for 4 hours to obtain the final product (a number average molecular weight was tested to be 80,000 to 100,000 by GPC).

Solution 4

12.0 g of ε-caprolactone, and 3.0 g of PEG-200 were respectively weighed and added into a vacuum reaction flask with stannous octoate as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 3 g of L-lysine diisocyanate and 0.8 g of 1,3-propanediol were weighed, added into a test tube, vacuumized and an opening of the test tube was sealed. The test tube was put into an oil bath pan at 70°C to react for 4 hours to obtain the final product (a number average molecular weight was tested to be 100,000 to 120,000 by GPC).

Solution 5

12.0 g of ε-caprolactone, and 3.0 g of PEG-600 were respectively weighed and added into a vacuum reaction flask with stannous octoate as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 3 g of L-lysine diisocyanate and 0.7 g of 1,3-propanediol were weighed, added into a test tube, vacuumized and an opening of the test tube was sealed. The test tube was put into an oil bath pan at 70°C to react for 10 hours to obtain the final product (a number average molecular weight was tested to be 120,000 to 150,000 by GPC).

Solution 6

19.0 g of ε-caprolactone, and 3.0 g of PEG-200 were respectively weighed and added into a vacuum reaction flask with stannous octoate as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 3.6 g of L-lysine diisocyanate and 1.56 g of 1,3-propanediol were weighed, added into a test tube, vacuumized and an opening of the test tube was sealed. The test tube was put into an oil bath pan at 70°C to react for 10 hours to obtain the final product (a number average molecular weight was tested to be 150,000 to 170,000 by GPC).

Solution 7

25.0 g of ε-caprolactone, and 3.0 g of PEG-600 were respectively weighed and added into a vacuum reaction flask with stannous octoate as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 4.3 g of L-lysine diisocyanate and 1.88 g of 1,3-propanediol were weighed, added into a test tube, vacuumized and an opening of the test tube was sealed. The test tube was put into an oil bath pan at 70°C to react for 10 hours to obtain the final product (a number average molecular weight was tested to be 160,000 to 180,000 by GPC).

Solution 8

35.0 g of ε-caprolactone, and 5.0 g of PEG-600 were respectively weighed and added into a vacuum reaction flask with stannous octoate as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 5.3 g of L-lysine diisocyanate and 1.7 g of 1,3-propanediol were weighed, added into a test tube, vacuumized and an opening of the test tube was sealed. The test tube was put into an oil bath pan at 70°C to react for 24 hours to obtain the final product (a number average molecular weight was tested to be 190,000 to 200,000 by GPC).

Solution 9

9.0 g of ε-caprolactone, and 3.0 g of PEG-400 were respectively weighed and added into a vacuum reaction flask with stannous octoate as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 3 g of L-lysine diisocyanate and 2.1 g of phenylalanine diamine bound by 1,3-propanediol through an ester bond were weighed, put into a vacuum reaction flask, vacuumized and then an opening of the vacuum reaction flask was sealed, and then the vacuum reaction flask was put into an oil bath pan at 60°C to react for 4 hours to obtain the final product (a number average molecular weight was tested to be 50,000 to 70,000 by GPC).

Solution 10

9.0 g of ε-caprolactone, 2.5 g of GA and 3.0 g of PEG-600 were respectively weighed and added into a vacuum reaction flask with stannous octoate (0.03wt%% of the total amount) as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 3 g of L-lysine diisocyanate and 2.6 g of indoline diamine bound by 1,3-propanediol through an ester bond

were weighed, put into a vacuum reaction flask, vacuumized and then an opening of the vacuum reaction flask was sealed, and then the vacuum reaction flask was put into an oil bath pan at 60°C to react for 4 hours to obtain the final product (a number average molecular weight was tested to be 60,000 to 80,000 by GPC).

Solution 11

12.0 g of ε-caprolactone, 3.0 g of LA, and 3.0 g of PEG-1000 were respectively weighed and added into a vacuum reaction flask with stannous octoate (0.03wt%% of the total amount) as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 4.3 g of L-lysine diisocyanate and 2.4 g of tryptophan diamine bound by 1,3-propanediol through an ester bond were weighed, put into a vacuum reaction flask, vacuumized and then an opening of the vacuum reaction flask was sealed, and then the vacuum reaction flask was put into an oil bath pan at 55°C to react for 4 hours to obtain the final product (a number average molecular weight was tested to be 100,000 to 120,000 by GPC).

Solution 12

9.0 g of ε-caprolactone, and 3.0 g of PEG-600 were respectively weighed and added into a vacuum reaction flask with stannous octoate (0.03wt%% of the total amount) as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 3 g of L-lysine diisocyanate and 0.9 g of phenylalanine diamine bound by 1,3-propanediol through an ester bond were weighed, put into a vacuum reaction flask, vacuumized and then an opening of the vacuum reaction flask was sealed, and then the vacuum reaction flask was put into an oil bath pan at 60°C to react for 4 hours to obtain the final product (a number average molecular weight was tested to be 60,000 to 80,000 by GPC).

Solution 13

8.0 g of ε-caprolactone, 6.0 g of PDO, and 4.0 g of PEG-1000 were respectively weighed and added into a vacuum reaction flask with stannous octoate (0.03wt%% of the total amount) as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 3 g of L-lysine diisocyanate and 0.7 g of 1,3-propanediol were weighed, put into a vacuum reaction flask, vacuumized and then an opening of the vacuum reaction flask was sealed, and then the vacuum reaction flask was put into an oil bath pan at 70°C to react for 4 hours to obtain the final product (a number average molecular weight was tested to be 50,000 to 70,000 by GPC).

Solution 14

12.0 g of ε-caprolactone, and 3.0 g of PEG-400 were respectively weighed and added into a vacuum reaction flask with stannous octoate (0.03wt%% of the total amount) as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 3 g of L-lysine diisocyanate and 0.55 g of 1,3-propanediol through an ester bond were weighed, put into a vacuum reaction flask, vacuumized and then an opening of the vacuum reaction flask was sealed, and then the vacuum reaction flask was put into an oil bath pan at 80°C to react for 4 hours to obtain the final product (a number average molecular weight was tested to be 100,000 to 120,000 by GPC).

Solution 15

9.00 g of ε-caprolactone, and 3.00 g of PEG-400 were respectively weighed and added into a vacuum reaction flask with stannous octoate (0.03wt%% of the total amount) as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 3 g of L-lysine diisocyanate and 0.5 g of 1,3-propanediol were weighed, and then added with 0.5 g of PEG-1500, and put into a vacuum reaction flask, vacuumized and then an opening of the vacuum reaction flask was sealed, and then the vacuum reaction flask was put into an oil bath pan at 70°C to react for 4 hours to obtain the final product (a number average molecular weight was tested to be 80,000 to 100,000 by GPC).

Solution 16

8.0 g of ε-caprolactone, and 4.0 g of PEG-200 were respectively weighed and added into a vacuum reaction flask with stannous octoate (0.03wt%% of the total amount) as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 4 g of L-lysine diisocyanate and 1.8 g of proline diamine bound by 1,3-propanediol through an ester bond were weighed, put into a vacuum reaction flask, vacuumized and then an opening of the vacuum reaction flask was sealed, and then the vacuum reaction flask was put into an oil bath pan at 70°C to react for 4 hours to obtain the final product (a number average molecular weight was tested to be 100,000 to 120,000 by GPC).

Solution 17

6.00 g of ε-caprolactone, and 6.00 g of PEG-400 were respectively weighed and added into a vacuum reaction flask with stannous octoate (0.03wt%% of the total amount) as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 6 g of L-lysine diisocyanate and 1.1 g of tyrosine diamine bound by 1,3-propanediol through an ester bond were weighed, put into a vacuum reaction flask, vacuumized and then an opening of the vacuum reaction flask was sealed, and then the vacuum reaction flask was put into an oil bath pan at 70°C to react for 10 hours to obtain the final product (a number average molecular weight was tested to be 120,000 to 150,000 by GPC).

Solution 18

8.5 g of ε-caprolactone, and 4.0 g of PEG-300 were respectively weighed and added into a vacuum reaction flask with stannous octoate (0.03wt%% of the total amount) as a catalyst, then a magnetic stirrer was added, vacuumized/filled with nitrogen for three times. Then, an opening of the vacuum reaction flask was sealed under vacuumizing, and the vacuum reaction flask was put into an oil bath pan at 140°C to react for 24 hours to obtain a linear polymer. 4 g of L-lysine diisocyanate and 1.8 g of glycine diamine were weighed, put into a vacuum reaction flask, vacuumized and then an opening of the vacuum reaction flask was sealed, and then the vacuum reaction flask was put into an oil bath pan at 70°C to react for 4 hours to obtain the final product (a number average molecular weight was tested to be 110,000 to 130,000 by GPC).

Embodiment 11: Degradation experiment of PLGA and PU ureteral stent with gradient degradation prepared by the same process

1. Preparation of a gradient degradable magnesium wire

Two one-meter-long magnesium wires (0.25 mm to 0.35 mm in diameter, and 0.12 mm in thickness) were completely soaked in a 15g/L dipotassium hydrogen phosphate aqueous solution containing 1.2 g/L phytic acid at about 60°C, and lifted by 5 cm every 2.5 minutes to form a gradient passivation protective film, one end passivated for short time was marked as B end and one end passivated for long time was marked as A end.

2. Preparation of a PU degradable ureteral stent

(1) the gradient degradable magnesium alloy wire (alloying constituent proportions were selected according to Table 9) processed was crimped into a single-strand spiral shape, with a pitch of 1.8 mm to 2.5 mm (as shown in FIG. 1) and a a total stent length of 26 cm to 28 cm;

(2) a degradable medical polyurethane material (polyurethane with PEG400 as an initiator, PCL as a soft segment and L-lysine diisocyanate as a hard segment, a number average molecular weight of 50,000 to 70,000 (referring to Solution 9 in Embodiment 10 for specific synthesis method), a breaking strength of 20 MPa, and an elongation at break of 250%) was dissolved in a chloroform solvent to prepare a 8%-15% solution for later use;

(3) the solution in step (2) was repeatedly dip-coated or sprayed on the stent in step (1) to manufacture the same into the coated stent (the stent had a tube wall thickness of 0.25 mm to 0.3 mm); and

(4) the composite material prepared in (3) was dip-coated or sprayed with a hydrophilic coating (such as aqueous solution made of PVA, PVP, and micromolecule hyaluronic acid, etc.), aired, polished and ground into the coated stent.

3. Preparation of a PLGA degradable ureteral stent

(1) the gradient degradable magnesium alloy wire (alloying constituent proportions were selected according to Table 9) processed was crimped into a single-strand spiral shape, with a pitch of 1.8 mm to 2.5 mm (as shown in FIG. 1) and a a total stent length of 26 cm to 28 cm;

(2) the PLGA (LA:GA=7:3) was dissolved in a chloroform solvate to prepare a 8%-15% solution solution for later use;

(3) the solution in step (2) was repeatedly dip-coated or sprayed on the stent in step (1) to manufacture the same into the coated stent (the stent had a tube wall thickness of 0.25 mm to 0.3 mm); and

(4) the composite material prepared in (3) was dip-coated or sprayed with a hydrophilic coating (such as aqueous solution made of PVA, PVP, and micromolecule hyaluronic acid, etc.), aired, polished and ground into the coated stent.

4. Preparation of a PU degradable ureteral stent

(1) a degradable medical polyurethane material (polyurethane with PEG400 as an initiator, PCL as a soft segment and L-lysine diisocyanate as a hard segment, a number average molecular weight of 50,000 to 70,000 (referring to Solution 9 in Embodiment 10 for specific synthesis method), a breaking strength of 20 MPa, and an elongation at break of 250%) was dissolved in a chloroform solvent to prepare a 8%-15% solution for later use; and
(2) the solution in (1) was repeatedly dip-coated or sprayed on a tetrafluoroethylene tube to manufacture the same into a PU tube (the tube had a wall thickness of 0.25 mm to 0.3 mm).

5. Product performances and comparative degradation experiments

The two ureteral stents prepared in this embodiment were soaked in artificial urine, and the artificial urine was changed every other week. After 20 days, it was found that flocculent degradation products began to appear on surfaces of the stent tubes, and then the surface degradation products became more and more; until 30 days, only the spiral magnesium wire remained, and then the magnesium wire gradually broke into fragments less than 2 mm from the B end to the A end; and after 40 days, the B end remained only was also degraded into debris.

| Name of stent | Main performances | | | Observation of degradation | | |
|---|---|---|---|---|---|---|
| | Hand feeling | Compressive resistance | Tensile strength | 20 days | 40 days | 60 days |
| PU coated stent | Soft | 3.5 N | 98% | Floccules began to adhere to the surface | Fine floccules dispersed in the degradation solution | The degradation products were in the form of fine debris |
| PLGA coated stent | Harder | 3.2 N | 30% | Tablets fell off | A number of tablets fell off | The degradation products became into large pieces |
| PU tube without magnesium wire | Soft | 0.1 N | 230% | Floccules began to adhere to the surface | Broken into multiple segments | The degradation products were small segments and tablets |

The above comparative research results show that: if only PU is used to prepare the stent tube, the compressive resistance is only 0.1 N, the stent tube is broken into multiple segments during the degradation process, and the final degradation fragments are also large, which are not suitable for human implantation. If PLGA is used as the coated stent, the degradation products of the stent are also tablets, which have a potential risk of blocking the ureter. The PU material developed by the disclosure has excellent controllable gradient degradability, which can meet the clinical needs and prepare innovative products meeting clinical use.

Embodiment 12:

The embodiments disclosed in CN105169496A and the embodiments of the disclosure were used to prepare PU by the same process, and influences of the degradation products on the proliferation of mouse fibroblasts in vitro were studied by MTT cell proliferation test. In the evaluation of biomaterial performances, the biocompatibility of the degradation products was a very important index, and usually the simplest and most direct method was to make a basic judgment on an inhibition rate of the degradation products to cells.

1. PU synthesis: sample 1: PU1 was synthesized according to the method of Embodiment 1 disclosed in CN105169496A, and PU2 was synthesized according to the method of Solution 1 in Embodiment 10 of the disclosure, molecular weights of which were both 50,000 to 70,000. Due to the substantially close feeding ratios, the main difference was that 1,4-butanediol was used as a chain extender in Embodiment 1 disclosed in CN105169496A, while 1,3-propanediol was used as a chain extender in Solution 1 in Embodiment 10 of the disclosure.

The specific experiments of PU1 and PU2 were as follows:

2. Preparation of degradation products: PU1 and PU2 were respectively purified to make various indexes thereof meet the technical requirements of biomaterials, 2 g of PU1 and 2 g of PU2 were put into a PBS buffer, degraded at 37°C for 30 days, then the degradation products were filtered, purified with chloroform, cleaned and dried to obtain white powder, and the white powder was extracted with normal saline. The samples obtained were processed as follows:

(1) sample PU1 (started from 400 ug/ml, twice diluted, and nine concentrations); and

(2) sample PU2 (started from 400 ug/ml, twice diluted, and nine concentrations).

3. Experimental method:

(1) cells were digested and counted to prepare a cell suspension of 7.5x104 cells/ml, and 100 μl of the cell suspension was added to each well of a 96-well cell culture plate;

(2) the 96-well cell culture plate was placed in a 5% $CO_2$ incubator at 37°C for 24 hours;

(3) the drug was diluted with a culture medium to a needed working solution concentration, 100 μl of corresponding drug-containing culture medium was added to each well, and a negative control group was set up at the same time;

(4) the 96-well cell culture plate was placed in a 5% $CO_2$ incubator at 37°C for 48 hours;

(5) the 96-well plate was stained with MTT, wherein λ=490 nm, and OD values were measured;
20 μl of MTT (5 mg/ml) was added to each well, and the culture was continued for 4 hours in the incubator;
a supernatant was discarded, 150 μl of DMSO was added to each well, shaken for 10 minutes and mixed gently;
λ=490nm, OD value of each well was read by a microplate reader, and an inhibition rate was calculated; and

(6) the inhibition rate of each group was calculated.

$$\text{Inhibition rate (\%)} = \frac{\text{OD value of negative control} - \text{OD value of experiment group}}{\text{OD value of experiment group}} \times 100\%$$

4. Experiment results: (refer to Excel for details)

| L929 48h | | | |
|---|---|---|---|
| Group | | Mean±SD | Inhibition rate |
| Negative control | | 0.629±0.017 | |
| PU1 IC50> 400ug/ml | 400ug/ml | 0.636±0.016 | -25.11% |
| | 200ug/ml | 0.631±0.005 | -13.32% |
| | 100ug/ml | 0.635±0.01 | -9.95% |
| | 50ug/ml | 0.628±0.005 | -6.16% |
| | 25ug/ml | 0.633±0.014 | -5.64% |
| | 12.5ug/ml | 0.636±0.005 | -3.11% |
| | 6.25ug/ml | 0.62±0.009 | 1.43% |
| | 3.13ug/ml | 0.633±0.009 | -0.64% |
| | 1.56ug/ml | 0.626±0.001 | 0.48% |

| L929 48h | | | |
|---|---|---|---|
| Group | | Mean±SD | Inhibition rate |
| Negative control | | 0.629±0.017 | |
| PU2 IC50> 400ug/ml | 800μM | 0.633±0.01 | -0.64% |
| | 400μM | 0.635±0.009 | -0.95% |
| | 200μM | 0.624±0.012 | 0.79% |
| | 100μM | 0.627±0.007 | 0.32% |
| | 50μM | 0.612±0.021 | 2.70% |
| | 25μM | 0.651±0.008 | -1.50% |
| | 12.5μM | 0.632±0.004 | -0.48% |
| | 6.25μM | 0.619±0.018 | 1.59% |
| | 3.13μM | 0.634±0.006 | -0.79% |

The experiment results show that: the degradation products of the PU obtained by using BDO as the chain extender have certain inhibitory effect on cell growth, showing certain cytotoxicity, while the PU obtained by using 1,3-propanediol has little inhibitory effect on cell growth. The reasons are analyzed as follows: a LD50 value (median lethal dose) of the 1,3-propanediol was 16,080 mg/kg (rats per os); and a LD50 value of the 1,4-butanediol is 1,525 mg/kg (rats per os). In terms of biosecurity, the 1,3-propanediol is one order of magnitude higher than the 1,4-butanediol. The PU of the disclosure uses the 1,3-propanediol instead of the 1,4-butanediol as the chain extender, which is basically free of cytotoxicity, while the cytotoxicity of using the 1, 4-butanediol as the chain extender is secondary. Therefore, through our continuous research on the synthesis of PU materials, the PU of the disclosure has outstanding advantages in the field of implantable medical appliances.

Embodiment 13: Preparation method of a drug-loading coated stent for preventing postoperative infection of intestinal tract (degradation time lasted for 3 months to 6 months)

(1) A magnesium alloy tube (alloying constituent proportions were selected according to Table 5) was cut into a spiral stent (the stent had an outer diameter of 5 mm which was 9 mm to 10 mm after expansion, and had a length of 3 cm to 5 cm), completely soaked in a 15% hydrofluoric acid solution for 10 minutes to form a passivation protective film, cleaned with acetone after the passivation was ended, and blow-dried for later use;

(2) a degradable medical polyurethane material (polyurethane with PEG200 as an initiator, PCL and PPDO as soft segments and L-lysine diisocyanate as a hard segment, a number average molecular weight of 160,000 to 180,000 (referring to Solution 7 in Embodiment 10 for specific synthesis method), a breaking strength of 30 MPa, and an elongation at break of 350%) was dissolved in in a chloroform solvent to prepare a 8%-15% solution for later use;

(3) an appropriate amount of antibiotics was dissolved or suspended in the solution in (2), and dip-coated or evenly sprayed on the stent in (1) to manufacture the same into the coated stent (the stent had a tube wall thickness of 0.4 mm); and

(4) the coated stent prepared in (3) was dip-coated or sprayed with a hydrophilic coating (such as aqueous solution made of PVA, PVP, and micromolecule hyaluronic acid, etc.), aired, polished and ground into the coated stent.

In conclusion, through repeated trials and screening, the disclosure screens out nontoxic biomaterials which can meet the requirements of stent preparation, so as to obtain better application in the fields of regenerative medicine and implanted materials.

The foregoing descriptions are merely embodiments of the disclosure, but are not intended to limit the patent scope of the disclosure. All equivalent structures or equivalent flow transformations made using the description of the disclosure and the accompanying drawings, or being used directly or indirectly in other related technical fields, are similarly included in the protection scope of the disclosure.

## Claims

1. A spiral coated stent with controllable gradient degradation, comprising a degradable medical polyurethane and a degradable magnesium alloy material, wherein a soft segment of the degradable medical polyurethane contains a following chemical structure: PCL-PEG-PCL, wherein a molecular weight of the PEG is 200 to 1,000, and a molecular weight of the PCL is 200 to 10,000;
the degradable magnesium alloy material is of a spiral structure; and
physical properties of the spiral coated stent with controllable gradient degradation need to satisfy the following technical parameters that:
a breaking strength needs to be no less than 1 N, a pressure resistance needs to be no less than 2 N, and a degradation characteristic of the magnesium alloy after surface treatment shows gradient degradation with different time after soaking in an aqueous solution.

2. The spiral coated stent with controllable gradient degradation according to claim 1, comprising the degradable medical polyurethane and the degradable magnesium alloy material, wherein a hard segment of the degradable medical polyurethane is L-lysine diisocyanate, and the soft segment contains the following chemical structure:

    PCL-PEG-PCL, wherein the molecular weight of the PEG is 200 to 1,000, and the molecular weight of the PCL is 200 to 5,000;
    the degradable magnesium alloy material is of a spiral structure; and
    the physical properties of the spiral coated stent with controllable gradient degradation need to satisfy the following technical parameters that:

        the breaking strength needs to be no less than 1 N, the pressure resistance needs to be no less than 2 N, and the degradation characteristic of the magnesium alloy after surface treatment shows gradient degradation with different time after soaking in the aqueous solution; and
        a weight percentage of the degradable medical polyurethane to the degradable magnesium alloy material is 10% to 99%: 1% to 90%.

3. The spiral coated stent with controllable gradient degradation according to claim 2, comprising the degradable medical polyurethane and the degradable magnesium alloy material, wherein the hard segment of the degradable medical polyurethane is L-lysine diisocyanate, and the soft segment contains the following chemical structure:

PCL-PEG-PCL, wherein the molecular weight of the PEG is 200 to 600, and the molecular weight of the PCL is 300 to 3,500; and a chain extender is selected from one of propylene glycol and diamine or diaminelike;
the degradable magnesium alloy material is of a spiral structure, which can be woven in a single-strand spiral or multi-strand spiral manner; and
the physical properties of the spiral coated stent with controllable gradient degradation need to satisfy the following technical parameters that:

the breaking strength needs to be no less than 1 N, an elongation at break needs to be no less than 50%, the pressure resistance needs to be no less than 2 N, and the degradation characteristic of the magnesium alloy after surface treatment shows gradient degradation with different time after soaking in an aqueous solution; and
the weight percentage of the degradable medical polyurethane to the degradable magnesium alloy material is 30% to 99%: 1% to 70%.

**4.** The spiral coated stent with controllable gradient degradation according to any one of claims 1 to 3, wherein the degradable polyurethane is prepared by one of the following preparation methods:

first method: using CL with different proportions and PEG with a molecular weight of 200 to 1,000 to synthesize a linear polycaprolactone diol, reacting the product with L-lysine diisocyanate, using propylene glycol or amino acid diamine as a chain extender and using organic tin or organic bismuth as a catalyst, and reacting to obtain the medical polyurethane used for the stent of the disclosure;
second method: using PDO and different diols to synthesize a linear PPDO poly diol, reacting the product with different diisocyanates, using different diols, amino acid diamines or diamines as chain extenders and using organic tin or organic bismuth as a catalyst, and reacting to obtain the medical polyurethane, which can be further added as a stent coating material in order to improve a degradation performance of the stent;
third method: using LA and GA with different molecular weights initiated by micromolecule diols for monomerization or copolymerization to obtain polymer diol, adipic acid polyester diol and oxalic acid polyester diol which are used as soft chains and reacted with LDI and different micromolecule diols or diamines, and using organic tin or organic bismuth as a catalyst, and reacting to obtain the medical polyurethane, which can be further added as a stent coating material in order to improve the degradation performance of the stent; and
fourth method: using hydroxyl-terminated polydimethylsiloxane as a soft segment and reacting with LDI and micromolecule diol or diamine, and using organic tin or organic bismuth as a catalyst to form an organosilicon-polyurethane block copolymer composed of softs segment and hard segments alternately, which can be further added as a stent coating material in order to improve the degradation performance of the stent.

**5.** The spiral coated stent with controllable gradient degradation according to any one of claims 1 to 3, wherein the degradable medical polyurethane material can further comprise the soft segment which is a polymer diol obtained by copolymerization of one or two of LA, GA, CL, PDO and adipic anhydride with a micromolecule diol as an initiator, and the chain extender is selected from micromolecule diol, diamine or diaminelike, and specifically selected from one or two of glycol, diglycol, tetraglycol, 1,3-propanediol, 1,4-butanediol, 1,6-hexanediol, 1,7-heptanediol, 1,8-octanediol, 1,9-nonanediol, 1,10-decanediol, ethylenediamine, propylene diamine, butanediamine, pentanediamine and amino acid diamine.

**6.** The spiral coated stent with controllable gradient degradation according to claim 1 or 2, comprising other polymer materials,
wherein hydrophobic polymer materials comprise: one or two of polylactic acid, polycaprolactone, poly(p-dioxanone) and copolymers thereof, polytrimethylene carbonate, polylactic acid-trimethylene carbonate copolymer, polycaprolactone-trimethylene carbonate copolymer, polyglycolic acid and polylactic acid-glycolic acid copolymer, and a viscosity-average molecular weight of the biodegradable polymer material is 500 to 1,000,000, which is used for adjusting one of a softness and a hardness of the material; and
hydrophilic polymer materials comprise: one of alginate, modified alginate and alginate degraded into hexosamine and N-acetylglucosamine, polyvinyl pyrrolidone series, starch grafted acrylonitrile, starch grafted hydrophilic monomer, polyacrylate, vinyl acetate copolymer, polyvinyl alcohol, modified polyvinyl alcohols, carboxymethyl cellulose, cellulose grafted acrylonitrile, cellulose grafted acrylate, cellulose xanthate grafted acrylate, cellulose grafted acrylamide, and epichlorohydrin cross-linked carboxymethyl cellulose; or one or a combination of a plurality of macromolecular antibacterial absorbent materials, various polyamino acids, chitosan and derivatives thereof, and polylysine.

7. The spiral coated stent with controllable gradient degradation according to claim 1 or 2, wherein the degradable magnesium alloy material is selected from materials refined from various chemical elements harmless to a human body, specifically comprising one or a combination of two of high-purity magnesium, magnesium-iron alloy, magnesium-zinc alloy, magnesium-calcium alloy, and magnesium-aluminum alloy, preferably high-purity magnesium and magnesium-zinc alloy such as Mg-Nd-Zn-Zr, Mg-Zn-Mn, Mg-Zn-Zr, Mg-Zn-Mn-Se-Cu, and Mg-Zn binary alloy.

8. The spiral coated stent with controllable gradient degradation according to any one of claims 1 to 3, comprising a contrast medium, specifically selected from one of zirconium dioxide, barium sulfate and iodine preparations.

9. A preparation method of a spiral coated stent with controllable gradient degradation, wherein, a first preparation method is as follows:

(1) preparing a gradient degradable magnesium wire: completely soaking a round wire or a flat wire of a one-meter-long magnesium wire in a dipotassium hydrogen phosphate aqueous solution containing phytic acid with a certain concentration or a 5%-30% hydrofluoric acid solution, and lifting by 1 cm to 10 cm every 1 minute to 10 minutes to form a gradient passivation protective film, marking one end passivated for short time as B end and marking one end passivated for long time as A end;
(2) crimping the degradable magnesium alloy wire processed in step (1) into a spiral pattern, dissolving a degradable medical polyurethane material or a composite material in an organic solvent to prepare a coating material, and evenly spraying the coating material on a surface of a stent through an electrostatic spinning nozzle in the continuous rotation process of the step (1) to manufacture the same into a coated composite stent with a thickness of 0.001 mm to 1 mm, and preferably 0.01 mm to 0.5 mm; and
(3) dissolving a hydrophilic material in water to prepare a required concentration, and dip-coating or evenly spraying on the surface of the stent to manufacture the same into a water-soluble coating, which is convenient for clinicians to place and use; and

a second preparation method is as follows:

(1) preparing a gradient degradable magnesium wire: completely soaking a round wire or a flat wire of a one-meter-long magnesium wire in a dipotassium hydrogen phosphate aqueous solution containing phytic acid with a certain concentration or a 5%-30% hydrofluoric acid solution, and lifting by 1 cm to 10 cm every 1 minute to 10 minutes to form a gradient passivation protective film, marking one end passivated for short time as B end and marking one end passivated for long time as A end;
(2) crimping the degradable magnesium alloy wire processed in step (1) into a spiral pattern, threading with tetrafluoroethylene or metal rod, spirally fixing the magnesium wire in a special grinding tool for manufacturing corrugated pipes according to a processing technology for manufacturing corrugated pipes, extruding the degradable medical polyurethane material with a tubule extruder to manufacture the same into a coated composite stent with a thickness of 0.001 mm to 1 mm, and preferably 0.01 mm to 0.5 mm; and
(3) dissolving a hydrophilic material in water to prepare a required concentration, and dip-coating or evenly spraying on the surface of the stent to manufacture the same into a water-soluble coating, which is convenient for clinicians to place and use.

10. An application of a spiral coated stent with controllable gradient degradation for preparing various tract stents in vivo, specifically comprising: blood vessel, vein, gullet, biliary tract, trachea, bronchus, small intestine, large intestine, urethra, ureter or other segments similar to a tubular passage, such as vascular stents, tracheal stents, bronchial stents, urethral stents, gullet stents, biliary stents, ureteral stents, ureteral stenosis stents, stents for small intestine, stents for large intestine, laryngeal implants, bypass catheters or ileostomy.

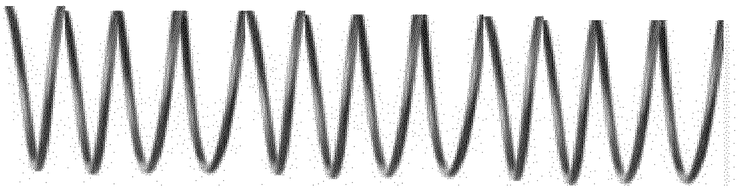

FIG. 1

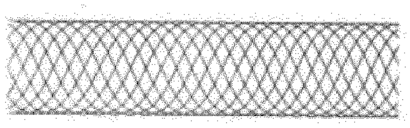

FIG. 2

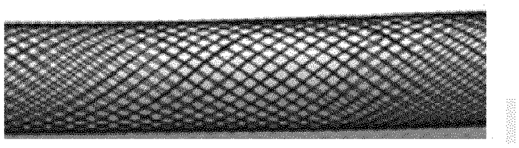

FIG. 3

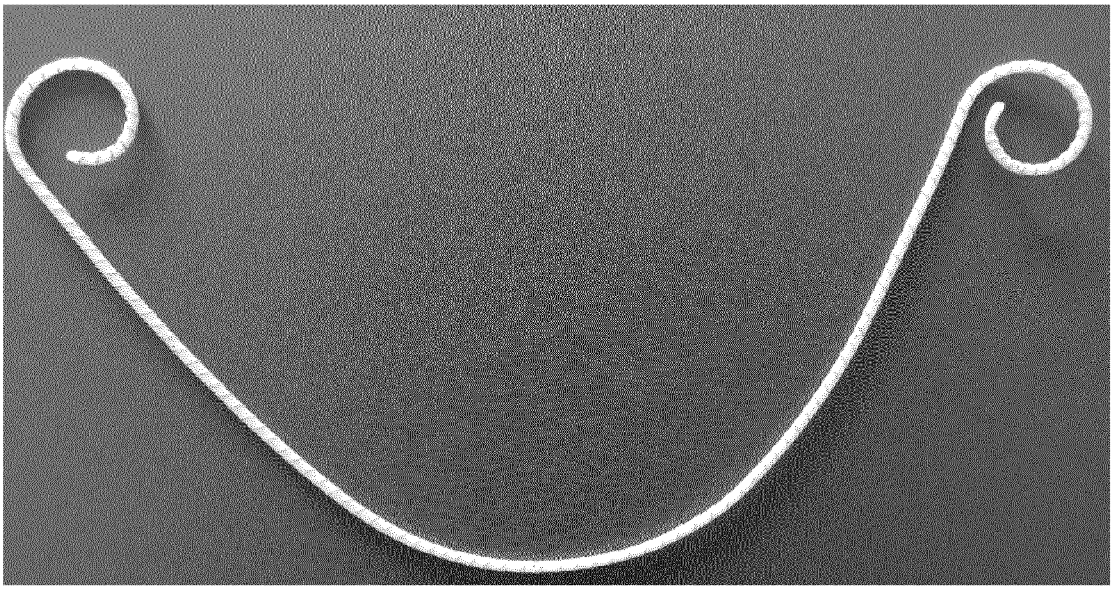

FIG. 4

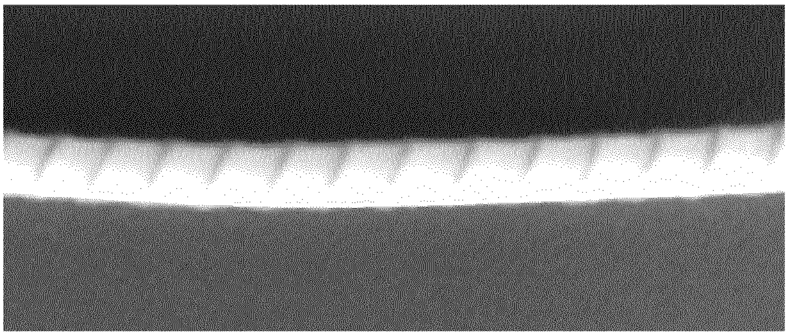

FIG. 5

**INTERNATIONAL SEARCH REPORT**

International application No.
**PCT/CN2018/106402**

**A. CLASSIFICATION OF SUBJECT MATTER**

A61L 31/02(2006.01)i

According to International Patent Classification (IPC) or to both national classification and IPC

**B. FIELDS SEARCHED**

Minimum documentation searched (classification system followed by classification symbols)

A61L

Documentation searched other than minimum documentation to the extent that such documents are included in the fields searched

Electronic data base consulted during the international search (name of data base and, where practicable, search terms used)

CNABS, VEN, ISI Web of Konwledge: 聚乙二醇, 支架, PEG, 聚己内酯, PCL, 凯斯蒂, 引发剂

**C. DOCUMENTS CONSIDERED TO BE RELEVANT**

| Category* | Citation of document, with indication, where appropriate, of the relevant passages | Relevant to claim No. |
|---|---|---|
| X | CN 105237714 A (POLYU BASE (SHENZHEN) LIMITED) 13 January 2016 (2016-01-13) claims 1-9, and description, paragraph 16 and embodiments 1-2 | 1-8 |
| X | CN 103767803 A (HU, KUN ET AL.) 07 May 2014 (2014-05-07) claim 6 | 9-10 |
| A | CN 103626979 A (EAST CHINA UNIVERSITY OF SCIENCE AND TECHNOLOGY) 12 March 2014 (2014-03-12) abstract, and claim 1 | 1-10 |
| A | CN 103073730 A (GRADUATE SCHOOL AT SHENZHEN, TSINGHUA UNIVERSITY) 01 May 2013 (2013-05-01) abstract, and claim 1 | 1-10 |
| A | CN 106110398 A (SOUTHWEST JIAOTONG UNIVERSITY) 16 November 2016 (2016-11-16) abstract, and claim 1 | 1-10 |
| A | WO 2016148648 A1 (NANYANG TECHNOLOGICAL UNIVERSITY ET AL.) 22 September 2016 (2016-09-22) abstract, and claim 1 | 1-10 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 March 2019** | **08 April 2019** |
| Name and mailing address of the ISA/CN<br>**State Intellectual Property Office of the P. R. China**<br>**No. 6, Xitucheng Road, Jimenqiao Haidian District, Beijing 100088**<br>**China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2018/106402**

| Patent document cited in search report | Publication date (day/month/year) | Patent family member(s) | Publication date (day/month/year) |
|---|---|---|---|
| CN 105237714 A | 13 January 2016 | None | |
| CN 103767803 A | 07 May 2014 | CN 106551731 A | 05 April 2017 |
| CN 103626979 A | 12 March 2014 | None | |
| CN 103073730 A | 01 May 2013 | CN 103073730 B | 22 July 2015 |
| CN 106110398 A | 16 November 2016 | None | |
| WO 2016148648 A1 | 22 September 2016 | CN 107920904 A | 17 April 2018 |
| | | SG 11201707686P A | 30 October 2017 |
| | | US 2018042742 A1 | 15 February 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 1950098 A
- CN 101636187 A
- CN 104744661 A
- CN 105169496 A